# EUROPÄISCHE PATENTSCHRIFT

(11) **EP 0 651 788 B1**
(45) Veröffentlichungstag und Bekanntmachung des Hinweises auf die Patenterteilung: **15.03.2000**
(21) Anmeldenummer: 93915821.8
(22) Anmeldetag: 07.07.1993
(51) Int. Cl.: C12N 5/08, G01N 33/50

(54) **VERFAHREN ZUM ZÜCHTEN EINER ZELLART IM KOKULTURVERFAHREN MIT LEBERZELLEN**
PROCESS FOR CO-CULTIVATING A TYPE OF CELLS WITH LIVER CELLS
PROCEDE DE COCULTURE D'UN TYPE DE CELLULES ET DE CELLULES HEPATIQUES

(30) Priorität: 08.07.1992 DE 4222345
(43) Veröffentlichungstag der Anmeldung: 10.05.1995
(73) Patentinhaber: BADER, Augustinus, D-31275 Lehrte (DE)
(72) Erfinder: BADER, Augustinus, D-31275 Lehrte (DE)
(74) Vertreter: Lorenz, Werner, Dipl.-Ing.
(86) Internationale Anmeldenummer: EP9301756
(87) Internationale Veröffentlichungsnummer: WO9401535

(56) Entgegenhaltungen:
- WO-A-84/04325
- ANNALS OF THE NEW YORK ACADEMY OF SCIENCES Bd. 349, 1980, NEW YORK Seiten 264 - 272 E. HUBERMAN ET AL. 'THE USE OF LIVER CELL CULTURES IN MUTAGENESIS STUDIES' DAS GANZE ARTIKEL

## Beschreibung

Die Erfindung betrifft ein Verfahren zur in vitro Herstellung hepatozellulärer Produkte und eine Ermöglichung der Einwirkung dieser Produkte auf eine zweite Zellart in einem stabilen zellulären Testsystem.

Bisher ist kein funktionell in vitro stabiles Testsystem für eukaryonte Zellen bekannt, in dem der Einfluß von Medikamenten oder chemischen Substanzen und zugleich auch deren Metaboliten auf eine beliebige Zellart hinsichtlich der Gesamtheit ihrer Wirkung untersucht werden konnte.

Ein Testsystem, das unter in vivo Bedingungen ablaufen könnte, hätte jedoch z.B. eine besondere Bedeutung für:
1. Die Entwicklung von Vorläufermedikamenten. Als Beispiel hierfür sind Prozytostatika zu nennen, die durch eine Metabolisierung, d.h. eine enzymatische Umwandlung in der Leber zu besser ausscheidbaren Substanzen, in ihre, für einen Tumor toxische Form übergeführt werden. Eine derartige, einem Patienten verabreichte Vorläuferform ist für den Gesamtorganismus nebenwirkungsarm. Dies bedeutet, derartige Substanzen stellen den Idealfall krebshemmender Medikamente dar.
2. Eine Identifizierung von Mutationen verursachenden Substanzen, oder Karzinogenen. Bekanntlich sind bestimmte in der Umwelt vorkommende Substanzen primär nicht krebserregend, werden aber nach Aufnahme in den menschlichen Organismus und nach erfolgter Metabolisierung aktiviert und damit krebserregend.
3. Zu Toxizitätsuntersuchungen. Bekanntlich müssen neuentwickelte Medikamente oder chemische Substanzen hinsichtlich einer potentiellen toxischen Wirkung untersucht werden. Eine Vielzahl von Medikamenten wirkt jedoch vor allem durch ihre Metabolisierung und eine speziesabhängig unterschiedlich ausgeprägte Entgiftungsfähigkeit toxisch.
4. Zur Untersuchung von Interaktionen. Darunter sind Wechselwirkungen zwischen verschiedenen Zellarten zu verstehen.

Bei den unter 1 bis 3 genannten Fällen handelt es sich insbesondere um Substanzen, die erst nach einer Metabolisierung in ihre aktive "Wirkform" übergeführt werden. Bekannt ist nun zu dessen Prüfung, die zu testenden Substanzen entweder Tieren zu verabreichen, oder nach einer Metabolisierung in einem Enzymgemisch auf bestimmte Zellarten zu applizieren oder auch in einem herkömmlichen Monolayerkokultursystem zwischen Leberzellen und einer weiteren Zellart zu untersuchen.

Nachteilig bei diesen bekannten Verfahren ist jedoch unter anderem, daß gravierende Unterschiede im Muster der Metabolitenentstehung nicht nur zwischen verschiedenen Tierarten sondern insbesondere auch zwischen Mensch und Tier vorhanden sind. Dies bedeutet, alle Substanzen, deren aktive oder toxische Form erst nach einer Metabolisierung in einem menschlichen Stoffwechselsystem entstehen können, sind in Tierversuchen nicht brauchbar zu ermitteln.

Aus diesen Grunde wurde bereits versucht, in Kokulturverfahren mit Leberzellen verschiedene Zellarten zu untersuchen (siehe z.B. WO 84/04325 und "Annals of the New York Academy of Sciences" 349, 1980, 264-272). Bei allen diesen bekannten Kokulturverfahren besteht jedoch das Problem, daß unmittelbar nach dem Ansetzen ein Funktionsverlust der Hepatozyten, d.h. der Leberzellen, in der Kultur einsetzt, da grundlegende hepatozytenspezifische und zellbiologische Anforderungen nicht berücksichtigt wurden und in der dabei verwandten Vorgehensweise nicht berücksichtigt werden konnten. Längerfristige Untersuchungen sind deswegen mit derartigen Systemen nicht sinnvoll. Diese wären jedoch sehr wichtig, denn bekanntlich zeigt sich die Eigenschaft von krebserregenden Stoffen erst nach einer längeren Zeit.

Bei den bekannten Kokulturverfahren wurden die Zellen in einer zweidimensionalen Schicht gemischt, wodurch sie nicht mehr auf einfache Weise selektiv getrennt werden konnten. Dies bedeutet, das Testsystem ist nicht wiederverwendbar.

Insbesondere wegen der auftretenden instabilen Funktion und deren nicht in vivo-artigen Reaktionsweisen der bekannten Testsysteme wurde auch bereits vorgeschlagen, Substanzen nach einer vorherigen Metabolisierung in einem Enzymgemisch auf die jeweiligen Zellarten zur Untersuchung eines Einflusses aufzutragen.

Nachteilig bei diesem Testsystem ist jedoch, daß wieder nur ein Teil der in vivo vorkommenden Metaboliten erfaßt werden kann, da eine komplette Metabolisierung an ein intaktes zelluläres System gebunden ist. Mögliche therapeutisch wirksame oder toxische Substanzen bleiben auf diese Weise unentdeckt, da sie überhaupt nicht entstehen können. Besonders nachteilig dabei ist jedoch, daß potentielle Karzinogene oder Zytostatika auf diese Weise nicht ermittelt werden können.

Eine Untersuchung von Interaktionen zwischen verschiedenen Zellarten, insbesondere zwischen Hepatozyten und einer weiteren Zellart in Kokultur, entsprechend Fall 4, wäre nur dann sinnvoll, wenn die Hepatozyten in der Lage wären, eine stabile Funktion in der Kultur zu behalten. Dies ist jedoch bei den bekannten Verfahren nicht der Fall.

Der vorliegenden Erfindung liegt daher die Aufgabe zugrunde, ein Verfahren zum Züchten einer Zellart im Kokulturverfahren zu schaffen, bei der über einen längeren Zeitraum wenigstens annähernd in vivo Bedingungen zur Untersuchung dieser Zellart erreicht werden können.

Erfindungsgemäß wird diese Aufgabe gelöst durch eine Kultivierung von Leberzellen auf einem Träger, wobei über den Leberzellen eine Matrixschicht angeordnet und darauf eine zweite zu untersuchende Zellenart aufgebracht wird.

Erfindungsgemäß wird in einer Art "Sandwichkulturtechnik" für Hepatozyten mit einem Anzüchten der zu untersuchenden zweiten Zellart oberhalb dieser Struktur in einem dreidimensionalen Kultursystem die vorstehend genannte Problematik gelöst.

Eines der Vorteile der erfindungsgemäßen Form des Kokulturverfahrens ist, daß auf diese Weise eine polarisierte Verankerung der Hepatozyten beibehalten wird.

Da normalerweise eine derartige Zellstapelung oder Zelldichte sehr schnell zu einem Sauerstoffmangel der unterhalb gelegenen Leberzellen führen würde, wird in einer sehr vorteilhaften und nicht naheliegenden Weiterbildung der Erfindung vorgeschlagen, daß über den Träger Sauerstoff in Form einer Membranoxygenierung zu den Leberzellen gebracht wird.

Auf diese Weise wird eine sogenannte Membranoxygenation erreicht, wenn der Träger entsprechend ausgebildet ist und die Leberzellen werden ausreichend mit Sauerstoff versorgt.

Praktisch liegt eine Kombination dreier Techniken vor, die es ermöglicht, die funktionellen Vorteile einer Sandwichleberzellkulturtechnik zur Herstellung hepatozellulärer Produkte und deren unmittelbare Einwirkung auf eine beliebig oberhalb davon kokultivierte Zellart auszunutzen und umgekehrt.

Das erfindungsgemäße Verfahren besteht somit aus drei zum Teil bekannten Techniken, nämlich einer Membranoxygenierung durch einen Träger, einem Sandwichverfahren für die Kultivierung von Leberzellen, und einem in dieser Form neuen Kokulturverfahren.

Durch dieses Verfahren schaltet man Oxygenationsprobleme aus, erhält eine stabile Leberzellenkultur und kann gleichzeitig eine Kokultivierung einer Zellart durchführen, durch die man Effekte untersuchen kann, die z.B. durch die Leberzellen und deren Metabolisierungskapazität erzeugt werden.

Im Gegensatz zu bekannten Testsystemen, insbesondere von bekannten Kokulturverfahren, liegt nunmehr erfindungsgemäß ein funktionell stabiles Testsystem vor, in dem Metaboliten über einen Zeitraum von mehreren Wochen hin produziert und in ihrer Wirkung untersucht werden können. Gleiches gilt auch für zelluläre Interaktionen. Auf diese Weise sind erstmals mittel- bis langfristige Toxizitätsuntersuchungen möglich.

Da bei einer Übereinanderanordnung von Leberzellen und der zur untersuchenden zweiten Zellart, deren Trennung z.B. durch eine Matrixschicht erfolgen kann, lassen sich bei Bedarf auch beide Zellarten wieder selektiv voneinander trennen. Dies kann z.B. durch Zugabe eines Enzymes zu der oberhalb liegenden kultivierten Zellart erfolgen. Dabei hat sich z.B. als geeignetes Enzym zur Ablösung der Zellen Trypsin herausgestellt.

Die längerfristige funktionelle Stabilität der Leberzellen und die Ablösbarkeit einer Zellart unter Erhalt der im Sandwichsystem immobilisierten Leberzellen ermöglicht auch eine Wiederverwendbarkeit des Testsystemes.

So kann z.B. eine andere, zu testende Zellart nach Waschen des Systemes erneut angezüchtet werden. Dies ist insbesondere deshalb von Bedeutung, weil humane Zellen, wie z.B. humane Leberzellen, nicht unbegrenzt erhältlich sind.

Ein weiterer sehr bedeutender Vorteil des erfindungsgemäßen Verfahrens liegt auch in der Verwendbarkeit menschlicher Hepatozyten, die damit eine sehr hohe Relevanz bezüglich ihrer Ergebnisse beim Menschen erreichen, was ja gerade das Ziel aller humanpharmazeutischen und humantoxikologischen Untersuchungen ist.

Eine höhere Relevanz liegt auch gegenüber Verfahren unter Verwendung von Enzymgemischen vor, da ja ein intaktes Zellsystem erfindungsgemäß vorliegt, und somit die Gesamtheit aller Metaboliten getestet werden kann. Bekannte Kokulturverfahren haben hier nur eine sehr geringe Relevanz für mittel- und längerfristige Untersuchungen, da ja die Leberzellen in derartigen bekannten Systemen sehr rasch metabolische Enzyme verlieren.

Nachfolgend ist ein Ausführungsbeispiel des erfindungsgemäßen Verfahrens prinzipmäßig näher erläutert.

Dargestellt ist dabei ein Verfahren zum Züchten einer Zellart im Kokulturverfahren mit übereinander angeordneten Zellarten.

Für eine Kokultivierung wird zuerst als Träger eine zumindest gaspermeable Membrane 1 mit einer Matrix 2 beschichtet. Als gaspermeable Membrane 1 kann z.B. ein Träger verwendet werden, der aus einem Material mit entsprechend großen Poren besteht oder Bohrungen versehen ist, durch die Sauerstoff geleitet wird bzw. diffundiert. Hierfür eignen sich z.B. Sintermetallscheiben, in die von einer Sauerstoffquelle aus Sauerstoff in Kanäle oder Bohrungen im Inneren der Scheibe eingeleitet wird, von wo aus der Sauerstoff über entsprechende Bohrungen oder Poren zur Oberfläche der Membrane 1 weitergeleitet wird. Ebenso sind poröse Kunststoffe oder Zellstoff als Membrane 1 geeignet.

Als Matrix 2 kann z.B. eine proteinhaltige Schicht verwendet werden, wie z.B. Kollagen. Dabei können gegebenenfalls weitere Komponenten wie z.B. Glycosaminoglykane oder Glykoprotein bei Bedarf hinzugemischt werden. Derartige Komponenten üben induktive Funktionen auf Hepatozyten aus. Hepatozyten, d.h. Leberzellen 3 werden polarisiert in bzw. über die Matrix 2 eingebettet.

Als Zellart zu Kokultur mit den Hepatozyten können z.B. V 79-Zellen oder Lymphozyten verwendet werden. Diese Zellen sind insbesondere für Mutagenitätsteste von Vorteil.

Die Matrix 2 dient auch der Adhäsionsverbesserung der Leberzellen 3. Gegebenenfalls könnten die Leberzellen 3 auch direkt auf die Membrane 1 aufgelegt werden, ohne die dazwischen liegende Matrix 2, wenn dieses Membran ein Anhaften der Leberzellen 3 ermöglicht, wie z.B. Polyurethanfolien, Polypropylen oder organische Membrane, wie z.B. menschliche oder tierische Bindegewebe.

Die Leberzellen 3 können in bekannter Weise mittels einer Kollagenaseverdauung des bindegewebigen Gerüstes einer Leber isoliert und dann gewaschen werden.

Nach einem Aufbringen der Leberzellen 3 auf die erste Matrixschicht 2 kann nach ca. 30 Minuten bis 1 stunde bei Verwendung von Kollagen als basale Adhäsionsstruktur mit dem Auftragen einer zweiten Matrixschicht 4 über den Leberzellen 3 begonnen werden. Die zweite Matrixschicht 4 dient der Verankerung der Leberzellen 3 mit ihren oben liegenden Zellmembranen an diese Struktur. Ebenso wie für die erste Matrixschicht 2 können gegebenenfalls weitere Substanzen der zweiten Matrixschicht 4 zugemischt werden.

Nach einer erfolgten Verfestigung der zweiten, oberen Matrixschicht 4 kann eine beliebige zweite, oder auch mehrere Zellarten 5, darüber aufgegeben werden.

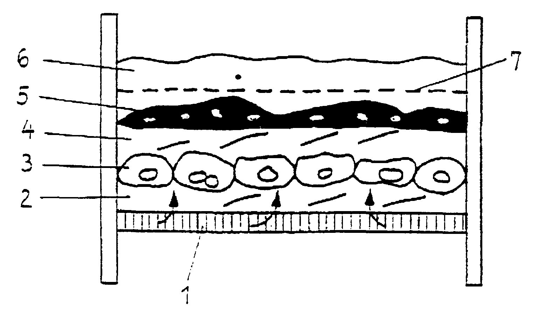

Abschließend wird Kultur- bzw. Nährmedium 6 in einer Schicht über die obere zu untersuchende Zellschicht 5 aufgebracht. Bei bestimmten beidseitig verankerungspflichtigen Zellarten 5 kann eine zusätzliche Anbringung einer dritten Matrixschicht über der zu untersuchenden Zellart 5 vorgesehen werden. Dies ist in der Figur durch die gestrichelte Linie 7 angedeutet. Auch in diesem Falle stellt die Schicht aus Kultur- bzw. Nährmedium 6 den oberen Abschluß über der dritten Matrixschicht 7 dar. Eine dritte Matrixschicht 7 wird im allgemeinen vorgesehen werden, wenn z.B. die oberen zu untersuchenden Zellen 5 ebenfalls Leberzellen sind.

Mit dem dargestellten Verfahren ist eine Kokultur von Leberzellen 3 mit einer beliebigen zweiten Zellart 5 möglich, und zwar unter Erhaltung eines längerfristig stabilen hepatozellulären Phänotypus.

Da die Sauerstoffversorgung der Leberzellen 3 mittels der Membranoxygenierung gewährleistet ist, kann jede beliebige andere Zellart in herkömmlicher Weise auf der Grundlage der zweiten oberen Kollagenschicht 4 angezüchtet werden. Die Versorgung der zu untersuchenden Zellart 5 erfolgt dabei von oben her über das Kultur- bzw. Nährmedium 6.

Mit dem Verfahren nach dem Ausführungsbeispiel sind z.B. Toxizitätstestungen von Pharmaka möglich, da Zellen von Zielorganen potentieller Toxizität mit einem humanen Stoffwechselsystem direkt kombiniert werden können. Dies bietet damit die Möglichkeit eines Screeningverfahrens, d.h. eine Reihenuntersuchung von mehreren Substanzen.

Ebenso können humanspezifische Stoffwechselprodukte, die im Tierversuch nicht erfaßt werden können, wie auch die verschiedensten anderen Zellsysteme mit diesem Verfahren untersucht werden. Darüber hinaus besteht damit die Möglichkeit, eine genaue Lokalisation toxischer Wirkungen nach Zellsystemen.

Im Bedarfsfalle kann das Verfahren auch in einen dynamischen Kulturmediumkreislauf eingeschlossen werden.

Statt dem Auftragen der zweiten Zellart 5, d.h. die in ihrer durch die Metaboliten in ihrer Beeinflussung zu untersuchenden Zellart, auf die dem Kulturmedium zugewandte Matrixschicht 4 oberhalb der Leberzellen 3, kann diese zweite Zellart 5 auch auf einen separaten Träger (nicht dargestellt) aufgebracht werden. Dieser Träger wird dann zusammen mit den darauf angeordneten Zellen mit dem metabolitenreichen Medium aus dem Leberzellsystem bespült. Eine Annährung des zweiten Trägers mit der zweiten Zellart 5 in der Weise, daß sich ein Spalt mit Kulturmedium zwischen beiden Systemen befindet, ist dann möglich.

Anstelle einer gaspermeablen Membrane 1 kann als Träger auch eine einfache nicht gaspermeable Platte verwendet werden. Zur ausreichenden Sauerstoffversorgung müßte dann allerdings die Untersuchung der Zellen in einem Inkubator vorgenommen werden, in dem der Sauerstoffpartialdruck entsprechend der darüberliegenden Zelldichte so weit erhöht bzw. eingestellt werden kann, daß die Leberzellen ausreichend mit Sauerstoff versorgt werden. In der Praxis wird man jedoch im allgemeinen aus Gründen der Einfachheit und der leichteren Durchführbarkeit eine gaspermeable Membrane verwenden.

## Patentansprüche

1. Verfahren zum Züchten einer Zellart im Kokulturverfahren mit Leberzellen,
**gekennzeichnet** durch eine Kultivierung von Leberzellen (3) auf einem Träger (1), wobei über den Leberzellen (3) eine Matrixschicht (4) angeordnet und darauf eine zweite zu untersuchende Zellenart (5) aufgebracht wird.

2. Verfahren nach Anspruch 1,
**dadurch gekennzeichnet,** daß über den Träger (1) Sauerstoff in Form einer Membranoxygenierung zu den Leberzellen (3) gebracht wird.

3. Verfahren nach Anspruch 1 oder 2,
**dadurch gekennzeichnet,** daß zwischen den Leberzellen (3) und dem Träger (1) eine erste Matrixschicht (2) zur Verankerung der Leberzellen (3) angeordnet wird.

4. Verfahren nach einem der Ansprüche 1 bis 3,
**dadurch gekennzeichnet,** daß über den zweiten Zellen (5), die über den Leberzellen (3) angeordnet werden, ein Kultur- oder Nährmedium (6) angeordnet wird.

5. Verfahren nach Anspruch 4,
**dadurch gekennzeichnet,** daß für die zweite Zellart (5) eine dritte Matrixschicht (7) angeordnet wird, über die das Kultur- bzw. Nährmedium (6) gelegt wird.

6. Verfahren nach einem der Ansprüche 1 bis 5,
**dadurch gekennzeichnet,** daß die zweite Zellart (5) auf einem eigenen zweiten Träger angeordnet wird, wobei ein Spalt mit Kultur- bzw. Nährmedium (6) zwischen den beiden Zellarten (3,5) eingestellt wird.

7. Verfahren nach Anspruch 1,
**dadurch gekennzeichnet,** daß es in einem Inkubator mit erhöhtem Sauerstoffpartialdruck durchgeführt wird.

8. Verwendung eines Verfahrens nach Anspruch 1 bis 7 zur Toxizitätsprüfung von zu untersuchenden Substanzen.

## Claims

1. Method for co-cultivating a cell type with liver cells,
**characterised by**
a cultivation of liver cells (3) on a support (1) above which a matrix layer (4) is arranged and above which a second cell type to be examined (5) is applied.

2. Method in accordance with claim 1,
**characterised by the fact** that oxygen is supplied to the liver cells (3) in the form of membrane oxygenation via the support (1).

3. Method in accordance with claim 1 or 2,
**characterised by the fact** that a first matrix layer (2) is arranged between the liver cells (3) and the support (1) to anchor the liver cells (3).

4. Method in accordance with any of the claims 1 to 3,
**characterised by the fact** that a culture or nutritive medium (6) is arranged above the second cells (5) that are arranged above the liver cells (3).

5. Method in accordance with claim 4,
**characterised by the fact** that a third matrix layer (7) is arranged for the second cell type (5), above which the culture or nutritive medium (6) is placed.

6. Method in accordance with any of the claims 1 to 5,
**characterised by the fact** that the second cell type (5) is arranged on its own second support, a gap with culture or nutritive medium (6) being adjusted between the two cell types (3,5).

7. Method in accordance with claim 1,
**characterised by the fact** that it is conducted in an incubator with increased partial oxygen pressure.

8. Use of a method in accordance with claims 1 to 7 for toxicity testing of substances to be examined.

## Revendications

1. Procédé de co-culture d'un type de cellules et de cellules hépatiques,
**caractérisé** par le fait que l'on cultive des cellules hépatiques (3) sur un support (1), une couche matricielle (4) étant disposée sur les cellules hépatiques (3) et un deuxième type de cellules à analyser (5) étant déposé sur cette dernière couche.

2. Procédé selon la revendication 1,
**caractérisé en ce que** l'on fournit de l'oxygène aux cellules hépatiques par l'intermédiaire du support (1) sous la forme d'une membrane oxygénante (3).

3. Procédé selon l'une des revendications 1 ou 2,
**caractérisé en ce que** l'on dispose une première couche matricielle (2) entre les cellules hépatiques (3) et le support (1), pour assurer un ancrage des cellules hépatiques (3).

4. Procédé selon l'une quelconque des revendications 1 à 3,
**caractérisé en ce que** l'on place sur les deuxièmes cellules (5), qui sont disposées sur les cellules hépatiques (3), un milieu de culture ou un milieu nutritif (6).

5. Procédé selon la revendication 4,
**caractérisé en ce qu'** audit deuxième type de cellules (5) est associée une troisième couche matricielle (7) sur laquelle est déposé le milieu de culture ou le milieu nutritif (6).

6. Procédé selon l'une quelconque des revendications 1 à 5,
**caractérisé en ce que** ledit deuxième type de cellules (5) est disposé sur un deuxième support, un espace comportant un milieu de culture ou un milieu nutritif (6) étant agencé pour séparer les deux types de cellules (3, 5).

7. Procédé selon la revendication 1,
**caractérisé en ce qu'** il est mis en oeuvre dans un incubateur sous une pression partielle d'oxygène élevée.

8. Utilisation d'un procédé selon l'une quelconque des revendications 1 à 7 pour le contrôle de la toxicité de substances à analyser.
